# EUROPÄISCHE PATENTSCHRIFT

(11) **EP 0 819 680 B1**
(45) Veröffentlichungstag und Bekanntmachung des Hinweises auf die Patenterteilung: **18.06.2003**
(21) Anmeldenummer: 97112147.0
(22) Anmeldetag: 16.07.1997
(51) Int. Cl.: C07D 239/52, C07C 67/36

(54) **Verfahren zur Herstellung von Pyrimidin-2-essigsäureestern**
Process for the preparation of esters of pyrimidine-2-acetic acid
Procédé de préparation d'esters d'acide pyrimidine-2-acétique

(30) Priorität: 18.07.1996 CH 179896
(43) Veröffentlichungstag der Anmeldung: 21.01.1998
(73) Patentinhaber: Lonza AG, 3930 Visp (CH)
(72) Erfinder: Bessard, Yves, Dr., 3960 Sierre (Kanton Wallis) (CH); Stucky, Gerhard, Dr., 3902 Brig-Glis (Kanton Wallis) (CH)
(74) Vertreter: Winter, Brandl, Fürniss, Hübner, Röss, Kaiser, Polte Partnerschaft

(56) Entgegenhaltungen:
- EP-A- 0 353 640
- US-A- 5 322 959
- A.WADA ET AL.: "A CONVENIENT SYNTHESIS OF METHYL(E)-BETA-PYRIMIDINYLACRYLATES." SYNTHESIS., 7.Juli 1986, STUTTGART DE, Seiten 555-556, XP002053509

## Beschreibung

Die vorliegende Erfindung betrifft ein Verfahren zur Herstellung von substituierten Pyrimidin-2-essigsäureestern durch Umsetzung von 2-(Halomethyl)pyrimidinen mit Kohlenmonoxid und einem Alkohol in Gegenwart eines Katalysators und einer Base.
Die erfindungsgemäss herstellbaren Ester besitzen die allgemeine Formel

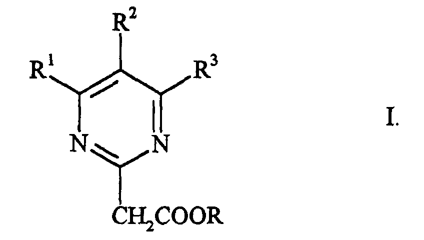

Hierin bedeuten:
RC₁₋₆-Alkyl, C₃₋₆-Cycloalkyl Fluorphenyl C₁₋₆-alkyl oder Aryl-C₁₋₆-alkyl, das gegebenenfalls am Arylrest mit C₁₋₆-Alkyl, halogeniert oder unhalogeniert, C₁₋₆-Alkoxy, C₁₋₆-Alkylthio und/oder C₁₋₆-Alkansulfonyl substituiert ist, wobei Aryl für Phenyl, Naphthyl, Biphenylyl oder Anthracenyl steht
R¹ bis R³ unabhängig voneinander Wasserstoff, C₁₋₆-Alkyl, fluoriertes C₁₋₆-Alkyl, C₁₋₆-Alkoxy, (C₁₋₆-Alkoxy)-C₁₋₆-alkyl oder (C₁₋₆-Alkoxy)carbonyl.

Verbindungen dieser Struktur sind Zwischenprodukte für die Herstellung von Herbiziden und Pflanzenwachstumsregulatoren (DE-A-38 26 230).

Eine bekannte Synthese dieser Verbindungen (mit R¹ = R³ = Alkoxy) geht von dem entsprechenden 2-(Chlormethyl)pyrimidin aus, das mit Natriumcyanid in das Pyrimidin-2-acetonitril übergeführt wird. Letzteres wird mit Alkohol/Chlorwasserstoff zum Iminoester-Hydrochlorid umgesetzt, welches anschliessend zum gewünschten Ester hydrolysiert wird (DE-A-38 26 230). Dieses Verfahren umfasst mehrere Stufen und hat eine nur mässige Ausbeute, ausserdem erfordert es den Umgang mit hochgiftigem Cyanid und korrosivem Chlorwasserstoff. US-A-5 322 959 offenbart die Carboxylierung von 1-Arylalkylhalogeniden in Anwesenheit eines Alkohols und eines Pd/Cu-Katalysators. A. Wada et al. (Synthesis 1986, S. 555-556) beschreiben ein Verfahren zur Herstellung von (E)-β-Pyrimidinylacrylaten unter Verwendung eines Palladiumdiacetat-triphenylphosphin-Komplexes.

Aufgabe der vorliegenden Erfindung war es, ein Verfahren zur Verfügung zu stellen, welches in nur einer Stufe mit guter Ausbeute das gewünschte Produkt liefert. Erfindungsgemäss wird diese Aufgabe durch das Verfahren nach Anspruch 1 gelöst.
Es wurde gefunden, dass 2-(Halomethyl)pyrimidine der allgemeinen Formel 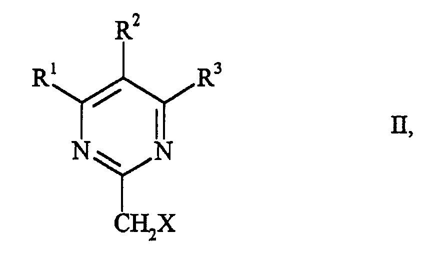 worin R¹ bis R³ die oben genannten Bedeutungen haben und X Chlor oder Brom ist, mit Kohlenmonoxid und einem Alkohol der allgemeinen Formel

R-OH III,

worin R die oben genannte Bedeutung hat, in Gegenwart einer Base aus der Gruppe, die aus den Alkali- und Erdalkalisalzen von Carbonsäuren mit 1 bis 6 Kohlenstoffatomen, den Alkali- und Erdalkalicarbonaten und -hydrogencarbonaten und den Alkali- und Erdalkali(hydrogen)phosphaten besteht, in guter Ausbeute direkt zu den gewünschten Produkten (I) reagieren, wenn als Katalysator ein Palladium-Phosphin-Komplex eingesetzt wird, in dem als Phosphin ein Diphosphin der allgemeinen Formel

R⁴R⁵P-Q-PR⁶R⁷ IV,

eingesetzt wird, worin R⁴ bis R⁷ unabhängig voneinander Phenyl, mit Fluor, C₁₋₆-Alkyl, halogeniert oder unhalogeniert, C₁₋₆-Alkoxy, C₁₋₆-Alkylthio und/oder C₁₋₆-Alkansulfonyl substituiertes Phenyl, C₁₋₆-Alkyl oder C₃₋₆-Cycloalkyl bedeuten und Q eine 1,1'-Ferrocendiylgruppe oder eine Gruppe der Formel -[CH₂]_{*n*}-, worin *n* 3 oder 4 ist, bedeutet.

Unter C₁₋₆-Alkyl sind hier und im folgenden alle linearen oder verzweigten primären, sekundären oder tertiären Alkylgruppen mit bis zu 6 Kohlenstoffatomen zu verstehen. Entsprechend sind unter C₁₋₆-Alkoxy und (C₁₋₆-Alkoxy)carbonyl die aus C₁₋₆-Alkyl und Sauerstoff bzw. Sauerstoff und Carbonyl zusammengesetzten Ether- und Esterfunktionen zu verstehen und analog dazu unter (C₁₋₆-Alkoxy)-C₁₋₆-alkyl die durch Austausch eines Wasserstoffatoms in C₁₋₆-Alkyl gegen C₁₋₆-Alkoxy gebildeten Alkoxyalkylgruppen, also beispielsweise Methoxymethyl oder Ethoxymethyl.
Phenyl-, Naphthyl-, Biphenylyl- oder Anthracenyl reste können einen oder mehrere gleiche oder verschiedene Substituenten tragen, nämlich C₁₋₆-Alkylgruppen wie Methyl, halogenierte C₁₋₆-Alkylgruppen wie Trifluormethyl, C₁₋₆-Alkoxygruppen wie Methoxy, oder C₁₋₆-Alkylthio-(Alkansulfanyl-) oder Alkansulfonylgruppen wie Methylthio oder Ethansulfonyl. Unter substituiertem Phenyl sind insbesondere Gruppen wie Fluorphenyl, Methoxyphenyl, Tolyl oder Trifluormethylphenyl zu verstehen, wobei sich die Substituenten vorzugsweise in *para*Stellung befinden.
Entsprechend sind unter Aryl-C₁₋₆-alkyl die aus C₁₋₆-Alkylgruppen durch Austausch eines Wasserstoffatoms gegen eine der vorstehend definierten Arylgruppen gebildeten Gruppen zu verstehen, also beispielsweise Benzyl oder Phenylethyl.

Die als Ausgangsmaterial dienenden 2-(Halomethyl)pyrimidine (II) sind bekannte Verbindungen oder können analog zu bekannten Verbindungen hergestellt werden, beispielsweise nach dem in EP-A-0 552 759 beschriebenen Verfahren.
Vorzugsweise werden als 2-(Halomethyl)pyrimidine die 2-(Chlormethyl)pyrimidine (X = Cl) eingesetzt.

Vorzugsweise werden nach dem erfindungsgemässen Verfahren C₁₋₄-Alkylester hergestellt (R = C₁₋₄-Alkyl), indem als Alkohol (III) das entsprechende C₁₋₄-Alkanol eingesetzt wird. Besonders bevorzugt sind Methyl-, Ethyl- und Isopropylester.

Ebenfalls bevorzugt ist die Herstellung von Pyrimidin-2-essigsäureestern (I), die in der Position 5 des Pyrimidinrings unsubstituiert sind (R² = H).

Besonders bevorzugt ist die Herstellung von Pyrimidin-2-essigsäureestern (I), die in den Positionen 4 und 6 des Pyrimidinrings (R¹, R³) Wasserstoff, C₁₋₄-Alkoxygruppen, (C₁₋₄-Alkoxy)carbonylgruppen oder (C₁₋₄-Alkoxy)methylgruppen tragen.

Als Phosphin im katalytisch aktiven Palladium-Phosphin-Komplex wird ein Diphosphin der allgemeinen Formel

R⁴R⁵P-Q-PR⁶R⁷ IV,

eingesetzt, worin R⁴ bis R⁷ unabhängig voneinander gegebenenfalls mit Fluor, C₁₋₆-Alkyl, halogeniert oder unhalogeniert, C₁₋₆-Alkoxy, C₁₋₆-Alkylthio und/oder C₁₋₆-Alkansulfonyl substituiertes Phenyl, C₁₋₆-Alkyl oder C₃₋₆-Cycloalkyl bedeuten und Q eine 1,1'-Ferrocendiylgruppe oder eine Gruppe der Formel -[CH₂]_{*n*}-, worin *n* 3 oder 4 ist, bedeutet.

Der katalytisch wirksame Palladium-Phosphin-Komplex wird vorteilhaft *in situ* gebildet, indem Palladium in fein verteilter elementarer Form (z. B. Palladium auf Aktivkohle), ein Pd(II) Salz (z. B. das Chlorid oder das Acetat), oder ein geeigneter Pd(II)-Komplex (z. B. Dichloro-bis(triphenylphosphin)palladium(II)) mit dem Phosphin zur Reaktion gebracht wird. Besonders bevorzugt sind Palladium(II)acetat und Dichloro-bis(triphenylphosphin)-palladium(II). Das Palladium wird vorzugsweise in einer Menge von 0,02 bis 2 Mol-% Pd(II) oder 0,5 bis 5 Mol-% Pd(0) (z. B. als Pd/C), jeweils bezogen auf die Halogenverbindung (II), eingesetzt. Das Phosphin wird vorteilhaft im Überschuss (bezogen auf Pd) eingesetzt, vorzugsweise in einer Menge von 0,2 bis 10 Mol-%, ebenfalls bezogen auf die Halogenverbindung (II).

Der Alkohol (III) kann auch gleichzeitig als Lösungsmittel dienen. Gegebenenfalls kann ein zusätzliches Lösungsmittel eingesetzt werden. Als zusätzliche Lösungsmittel kommen sowohl relativ unpolare, wie beispielsweise Toluol oder Xylol, als auch polare wie beispielsweise Acetonitril, Tetrahydrofuran oder *N,N*-Dimethylacetamid in Frage.

Als Base wird eine schwache Base aus der Gruppe der Alkali- oder Erdalkalicarbonate, der Alkali- oder Erdalkalisalze von niedrigen Carbonsäuren, der Alkali- oder Erdalkalihydrogencarbonate oder der Alkali- oder Erdalkali(hydrogen)phosphate eingesetzt. Besonders bevorzugt sind Alkalicarbonate und -acetate, insbesondere Natrium- und Kaliumcarbonat und -acetat.

Die Reaktionstemperatur liegt vorzugsweise bei 80 bis 250 °C.

Der Kohlenmonoxiddruck liegt vorzugsweise bei 1 bis 50 bar.

Die Reaktionsdauer hängt unter anderem von der Temperatur, der Reaktivität der eingesetzten Verbindungen und den Konzentrationsverhältnissen ab, sie liegt typischerweise im Bereich von einigen Stunden. Da bei übermässig langer Reaktionsdauer unter Umständen Folgereaktionen eintreten, wird der Reaktionsverlauf vorteilhaft mit einer geeigneten Analysenmethode (z. B. GC) überwacht und die Reaktion nach Erreichen der maximalen Produktkonzentration abgebrochen.

Die folgenden Beispiele verdeutlichen die Durchführung des erfindungsgemässen Verfahrens.

### Beispiel 1

### 4,6-Dimethoxypyrimidin-2-essigsäuremethylester (I, R = Me, R¹ = R³ = OMe, R² = H)

In einem indirekt beheizten (Ölbad) Metallautoklaven wurden 1,89 g (10 mmol) 2-(Chlormethyl)-4,6-dimethoxypyrimidin, 166 mg (0,3 mmol) 1,1'-Bis(diphenylphosphino)ferrocen, 4,5 mg (20 µmol) Palladium(II)acetat, 1,23 g (15 mmol) Natriumacetat und 40 ml Methanol vorgelegt. Der Autoklav wurde mehrmals mit Kohlenmonoxid gespült, dann wurde der Kohlenmonoxiddruck auf 15 bar erhöht und das Reaktionsgemisch 2 h bei 140 °C Badtemperatur/123 °C Innentemperatur erhitzt. Eine GC-Analyse des Reaktionsgemischs ergab eine Ausbeute von 65% bei einem Umsatz von 100%. Zur Aufarbeitung wurde das Reaktionsgemisch im Vakuum eingeengt und der Rückstand an Kieselgel 60 mit Hexan/Ethylacetat (3:1) chromatographiert.
- Isolierte Ausbeute:: 1,30 g (59%) gelbes Öl, Gehalt (GC) 96,5%
- ¹H NMR (CDCl₃) δ =: 5,92 (s, 1H); 3,92 (s, 6H); 3,82 (s, 2H); 3,75 (s, 3H).
- MS (*m*/*z*):: 212 (M⁺); 211; 183; 169; 140.

### Beispiel 2

### 4,6-Dimethoxypyrimidin-2-essigsäureethylester (I, R = Et, R¹ = R³ = OMe, R² = H)

Es wurde verfahren wie in Beispiel 1 beschrieben, jedoch wurde anstelle von Methanol Ethanol und anstelle von Natriumacetat 1,17 g (11 mmol) Natriumcarbonat eingesetzt. Die Badtemperatur betrug 140 °C, die Innentemperatur 127 °C und die Reaktionsdauer 2 h. Eine GC-Analyse des Reaktionsgemischs ergab eine Ausbeute von 97% bei 100% Umsatz.
- Isolierte Ausbeute:: 1,90 g (82,4%) gelbes Öl, Gehalt (GC) 98,1%
- ¹H NMR (CDCl₃) δ =: 5,92 (s, 1H); 4,22 (q, 2H); 3,92 (s, 6H); 3,80 (s, 2H); 1,28 (t, 3H).
- ¹³C NMR (CDCl₃) δ =: 14,2 (CH₂CH₃); 45,4 (CH₂CO); 54,0 (OCH₃); 60,9 (OCH₂); 87,9 (CH); 163,7 (C=O), 167,7 (N=C-N); 171,6 (*C*-OCH₃).
- MS (*m*/*z*):: 226 (M⁺); 211; 196; 181; 153; 122.

### Beispiel 3

### 4,6-Dimethoxypyrimidin-2-essigsäureisopropylester (I, R = i-Pr, R¹ = R³ = OMe, R² = H)

Es wurde verfahren wie in Beispiel 2 beschrieben, jedoch wurde anstelle von Ethanol Isopropylalkohol eingesetzt. Die Badtemperatur betrug 143 °C, die Innentemperatur 130 °C und die Reaktionsdauer 2 h. Eine GC-Analyse des Reaktionsgemischs ergab eine Ausbeute von 94% bei 100% Umsatz.
- Isolierte Ausbeute:: 1,34 g (54%) gelbes Öl, Gehalt (GC) 97%
- ¹H NMR (CDCl₃) δ =: 5,92 (s, 1H); 5,10 (sept., 1H); 3,91 (s, 6H); 3,78 (s, 2H); 1,26 (t, 6H).
- ¹³C NMR (CDCl₃) δ =: 21,81 (CH(CH₃)₂); 45,63 (CH₂CO); 54,02 (OCH₃); 68,31 (OCH); 87,91 (C-*C*H-C); 163,87 (C=O), 169,22 (N=C-N); 171,56 (*C*-OCH₃).
- MS (*m*/*z*):: 240 (M⁺); 197; 181; 154; 125; 113.

## Patentansprüche

1. Verfahren zur Herstellung von Pyrimidin-2-essigsäureestem der allgemeinen Formel 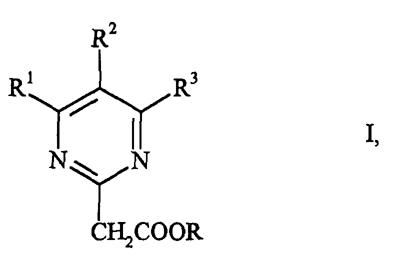 worin RC₁₋₆-Alkyl, C₃₋₆-Cycloalkyl, Fluorphenyl C₁₋₆-alkyl oder gegebenenfalls am Arylrest mit C₁₋₆-Alkyl, halogeniert oder unhalogeniert, C₁₋₆-Alkoxy, C₁₋₆-Alkylthio und/oder C₁₋₆-Alkansulfonyl substituiertes Aryl-C₁₋₆-alkyl ist, wobei Aryl für Phenyl, Naphthyl, Biphenylyl oder Anthracenyl steht, und R¹ bis R³ unabhängig voneinander Wasserstoff, C₁₋₆-Alkyl, fluoriertes C₁₋₆-Alkyl, C₁₋₆-Alkoxy, (C₁₋₆-Alkoxy)-C₁₋₆-alkyl oder (C₁₋₆-Alkoxy)carbonyl bedeuten, **dadurch gekennzeichnet, dass** ein 2-(Halomethyl)pyrimidin der allgemeinen Formel 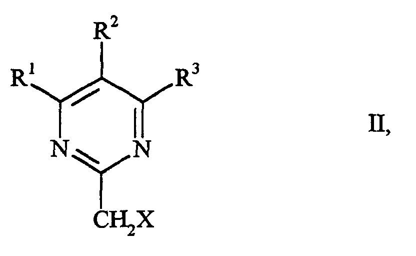 worin R¹ bis R³ die oben genannten Bedeutungen haben und X Chlor oder Brom ist, mit Kohlenmonoxid und einem Alkohol der allgemeinen Formel
R-OH III,
worin R die oben genannte Bedeutung hat, in Gegenwart eines katalytisch aktiven Palladium-Phosphin-Komplexes, in dem als Phosphin ein Diphosphin der allgemeinen Formel
R⁴R⁵P-Q-PR⁶R⁷ IV,
eingesetzt wird, worin R⁴ bis R⁷ unabhängig voneinander Phenyl, mit Fluor, C₁₋₆-Alkyl, halogeniert oder unhalogeniert, C₁₋₆-Alkoxy, C₁₋₆-Alkylthio und/oder C₁₋₆-Alkansulfonyl substituiertes Phenyl, C₁₋₆-Alkyl oder C₃₋₆-Cycloalkyl bedeuten und Q eine 1,1'-Ferrocendiylgruppe oder eine Gruppe der Formel -[CH₂]_{*n*}-, worin *n* 3 oder 4 ist, bedeutet, und einer Base aus der Gruppe, die aus den Alkali- und Erdalkalisalzen von Carbonsäuren mit 1 bis 6 Kohlenstoffatomen, den Alkali- und Erdalkalicarbonaten und -hydrogencarbonaten und den Alkali- und Erdalkali(hydrogen)phosphaten besteht, zur Reaktion gebracht wird.

2. Verfahren nach Anspruch 1, **dadurch gekennzeichnet, dass** X Chlor ist.

3. Verfahren nach Anspruch 1 oder 2, **dadurch gekennzeichnet, dass** R C₁₋₄-Alkyl ist.

4. Verfahren nach einem der Ansprüche 1 bis 3, **dadurch gekennzeichnet, dass** R² Wasserstoff ist.

5. Verfahren nach einem der Ansprüche 1 bis 4, **dadurch gekennzeichnet, dass** R¹ und R³ Wasserstoff, C₁₋₄-Alkoxy, (C₁₋₄-Alkoxy)carbonyl, oder (C₁₋₄-Alkoxy)methyl sind.

6. Verfahren nach einem der Ansprüche 1 bis 5, **dadurch gekennzeichnet, dass** der katalytisch aktive Palladium-Phosphin-Komplex *in situ* aus dem Phosphin und Palladium(II)acetat oder Dichloro-bis(triphenylphosphin)palladium(II) gebildet wird.

7. Verfahren nach einem der Ansprüche 1 bis 6, **dadurch gekennzeichnet, dass** als Base ein Alkalicarbonat oder -acetat, vorzugsweise Natriumcarbonat, Kaliumcarbonat, Natriumacetat oder Kaliumacetat, eingesetzt wird.

## Claims

1. Process for the preparation of pyrimidine-2-acetic acid esters of the general formula 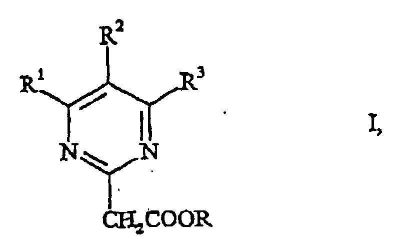 wherein R is C₁₋₆-alkyl, C₃₋₆-cycloalkyl, fluorophenyl-C₁₋₆-alkyl, or aryl-C₁₋₆-alkyl optionally substituted on the aryl radical by C₁₋₆-alkyl, halogenated or unhalogenated, C₁₋₆-alkoxy, C₁₋₆-alkylthio and/or by C₁₋₆-alkanesulfonyl, wherein aryl represents phenyl, naphthyl, biphenylyl or anthracenyl, and R¹ to R³ each independently of the others represents hydrogen, C₁₋₆-alkyl, fluorinated C₁₋₆-alkyl, C₁₋₆-alkoxy, (C₁₋₆-alkoxy)-C₁₋₆-alkyl or (C₁₋₆-alkoxy)carbonyl, **characterised in that** a 2-(halomethyl)pyrimidine of the general formula 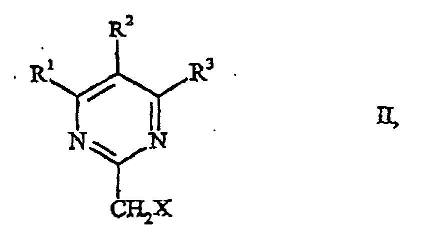 wherein R¹ to R³ are as defined above and X is chlorine or bromine, is reacted with carbon monoxide and an alcohol of the general formula
R-OH III,
wherein R is as defined above, in the presence of a catalytically active palladium-phosphine complex, in which there is used as the phosphine a diphosphine of the general formula
R⁴R⁵P-Q-PR⁶R⁷ IV,
wherein R⁴ to R⁷ each independently of the others represents phenyl, phenyl substituted by fluorine, C₁₋₆-alkyl, halogenated or unhalogenated, C₁₋₆-alkoxy, C₁₋₆-alkylthio and/or by C₁₋₆-alkanesulfonyl, C₁₋₆-alkyl or C₃₋₆-cycloalkyl, and Q represents a 1,1'-ferrocenediyl group or a group of the formula -[CH₂]_{*n*}-, wherein *n* is 3 or 4, and in the presence of a base from the group consisting of the alkali and alkaline earth salts of carboxylic acids having from 1 to 6 carbon atoms, the alkali and alkaline earth carbonates and hydrogen carbonates and the alkali and alkaline earth (hydrogen) phosphates.

2. Process according to claim 1, **characterised in that** X is chlorine.

3. Process according to either claim 1 or claim 2, **characterised in that** R is C₁₋₄-alkyl.

4. Process according to any one of claims 1 to 3, **characterised in that** R² is hydrogen.

5. Process according to any one of claims 1 to 4, **characterised in that** R¹ and R³ are hydrogen, C₁₋₄-alkoxy, (C₁₋₄-alkoxy)carbonyl or (C₁₋₄-alkoxy)methyl.

6. Process according to any one of claims 1 to 5, **characterised in that** the catalytically active palladium-phosphine complex is formed in *situ* from the phosphine and palladium(II) acetate or dichloro-bis(triphenylphosphine)-palladium(II).

7. Process according to any one of claims 1 to 6, **characterised in that** there is used as the base an alkali carbonate or acetate, preferably sodium carbonate, potassium carbonate, sodium acetate or potassium acetate.

## Revendications

1. Procédé de fabrication d'esters d'acide pyrimidine-2-acétique de formule générale 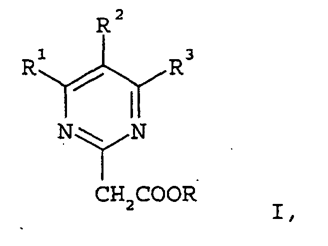 dans laquelle R est un groupe alkyle en C₁₋₆, un groupe cycloalkyle en C₃₋₆, un groupe fluorophénylalkyle en C₁₋₆ ou un groupe arylalkyle en C₁₋₆ substitué le cas échéant sur le radical aryle par un groupe alkyle en C₁₋₆, halogéné ou non halogéné, un groupe alkoxy en C₁₋₆, un groupe thioalkyle en C₁₋₆ et/ou un groupe alcanesulfonyle en C₁₋₆, aryle signifiant phényle, naphtyle, biphényle ou anthracyle, et R¹ à R³ représentant, indépendamment les uns des autres, un hydrogène, un groupe alkyle en C₁₋₆, un groupe alkyle en C₁₋₆ fluoré, un groupe alkoxy en C₁₋₆, un groupe (alkoxy en C₁₋₆)-alkyle en C₁₋₆ ou un groupe (alkoxy en C₁₋₆) carbonyle, **caractérisé en ce qu'**une 2-(halométhyl)pyrimidine de formule générale 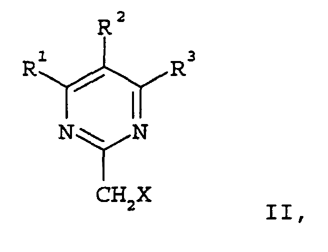 dans laquelle R¹ à R³ ont les significations citées ci-dessus et X est le chlore ou le brome, est mise à réagir avec du monoxyde de carbone et un alcool de formule générale
R-OH III,
dans laquelle R a la signification citée ci-dessus, en présence d'un complexe phosphine-palladium catalytiquement actif, dans lequel est mise en oeuvre en tant que phosphine une diphosphine de formule générale
R⁴R⁵P-Q-PR⁶R⁷ IV,
dans laquelle R⁴ à R⁷ représentent indépendamment les uns des autres, un groupe phényle, un groupe phényle substitué par du fluor, par un groupe alkyle en C₁₋₆ halogéné ou non halogéné, par un groupe alcoxy en C₁₋₆, par un groupe thioalkyle en C₁₋₆ et/ou par un groupe alcanesulfonyle en C₁₋₆, un groupe alkyle en C₁₋₆ ou un groupe cycloalkyle en C₃₋₆ et Q représente un groupe 1,1'-ferrocènediyle ou un groupe de formule -[CH₂]ₙ-, dans laquelle n est 3 ou 4, et une base du groupe constitué par les sels alcalins et alcalinoterreux d'acides carboxyliques avec de 1 à 6 atomes de carbone, les carbonates et hydrogénocarbonates alcalins et alcalinoterreux et les hydrogénophosphates alcalins et alcalinoterreux.

2. Procédé selon la revendication 1, **caractérisé en ce que** X est le chlore.

3. Procédé selon la revendication 1 ou 2, **caractérisé en ce que** R est un groupe alkyle en C₁₋₄.

4. Procédé selon l'une des revendications 1 à 3, **caractérisé en ce que** R² est l'hydrogène.

5. Procédé selon l'une des revendications 1 à 4, **caractérisé en ce que** R¹ et R³ sont un hydrogène, un groupe alkoxy en C₁₋₄, un groupe (alkoxy en C₁₋₄)-carbonyle, ou un groupe (alkoxy en C₁₋₄)méthyle.

6. Procédé selon l'une des revendications 1 à 5, **caractérisé en ce que** le complexe phosphine-palladium catalytiquement actif est formé *in situ* à partir de la phosphine et de l'acétate de palladium(II) ou du dichloro-bis(triphénylphosphine)palladium(II).

7. Procédé selon l'une des revendications 1 à 6, **caractérisé en ce qu'**on utilise comme base un carbonate ou un acétate alcalin, de préférence du carbonate de sodium, du carbonate de potassium, de l'acétate de sodium ou de l'acétate de potassium.
